# EUROPÄISCHE PATENTANMELDUNG

(11) **EP 4 074 816 A1**
(43) Veröffentlichungstag der Anmeldung: **19.10.2022**
(21) Anmeldenummer: 22163159.1
(22) Anmeldetag: 21.03.2022
(51) Int. Cl.: C12M 1/42, A23C 9/00

(54) **VERFAHREN UND VORRICHTUNG ZUR ELEKTROPORATION EINES BIOLOGISCHEN PROZESSGUTES**

(30) Priorität: 22.03.2021 DE 102021107046
(71) Anmelder: Elea Service GmbH, 49610 Quakenbrück (DE)
(72) Erfinder: Siemer, Claudia, 49593 Bersenbrück (DE); Toepfl, Stefan, 49076 Osnabrück (DE)
(74) Vertreter: Grünecker Patent- und Rechtsanwälte PartG mbB

(57) **Zusammenfassung**

Die vorliegende Erfindung betrifft ein Verfahren zum Elektroporieren eines biologischen Prozessgutes (1), wobei das biologische Prozessgut (1) elektroporiert wird, indem es einem niederfrequenten elektrischen Feld ausgesetzt wird, und eine Vorrichtung (20) zur Elektroporation eines biologischen Prozessgutes (1), umfassend einen Kondensator (3) zur Erzeugung eines niederfrequenten elektrischen Feldes in einem Behandlungsbereich (4). Um eine unerwünschte Befeuchtung oder Kontamination zu vermeinden, ist erfindungsgemäß vorgesehen, dass das dem elektrischen Feld ausgesetzte biologische Prozessgut (1) von einer Barriere (2) umschlossen ist bzw. dass eine Barriere (2) zur Aufnahme des biologischen Prozessgutes (1) in dem Behandlungsbereich (4) angeordnet ist, wobei die Barriere (2) im Wesentlichen flüssigkeitsundurchlässig und für das elektrische Feld durchlässig ist.

## Beschreibung

Die vorliegende Erfindung betrifft ein Verfahren zum Elektroporieren eines biologischen Prozessgutes, bei dem das biologische Prozessgut einem elektrischen Feld ausgesetzt wird.

Die vorliegende Erfindung betrifft ferner eine Vorrichtung zur Elektroporation von biologischem Prozessgut, die einen Kondensator zur Erzeugung eines elektrischen Feldes in einem Behandlungsbereich umfasst

Die vorliegende Erfindung betrifft außerdem ein elektroporiertes biologisches Prozessgut.

Elektroporation ist eine Methode, Zellmembranen permeabel zu machen, um so den Massentransport in die Zelle hinein oder aus der Zelle heraus zu verbessern. Die Elektroporation wird in der Molekularbiologie beispielsweise zum Transfer von Nukleinsäuren in prokaryotische oder eukaryotische Zellen eingesetzt. In der Lebensmittel- und Bioverfahrenstechnik wird die Elektroporation zur Verbesserung von Massentransportprozessen oder zur Inaktivierung von Mikroorganismen angewendet. Für die Elektroporation werden niederfrequente elektrische Felder von unter 9 kHz eingesetzt. Im Gegensatz zur Nutzung hochfrequenter Wechselfelder, wie etwa bei Mikrowellen, ist eine Übertragung der Energie niederfrequenter elektrischer Felder über ein Dielektrikum wie Luft nicht möglich. Es bedarf für die Übertragung der Energie niederfrequenter Felder entweder eines direkten Kontaktes des zu elektroporierenden Prozessgutes zwischen den mindestens zwei Elektroden eines Kondensators oder eines energieübertragenden Mediums, in der Regel Prozesswasser.

Der Einsatz energieübertragender Medien hat den Nachteil einer möglichen Kontamination des Prozesswassers durch Auslaugen oder Abwaschen von Produktbestandteilen des biologischen Prozessgutes. Des Weiteren kann Prozesswasser zu einer nachteiligen Befeuchtung oder Wasseraufnahme des Prozessgutes führen. Schließlich kann es zu einer unerwünschten Kontamination des Prozessgutes durch Kontakt zum Prozessmedium oder dessen Inhaltsstoffen, beispielsweise Hilfsstoffen oder Verschmutzungen kommen.

Es ist somit die Aufgabe der vorliegenden Erfindung, eine unerwünschte Befeuchtung oder Kontamination des biologischen Prozessgutes bzw. Prozesswassers zu vermeiden und gleichzeitig eine effektive Elektroporation zu ermöglichen.

Diese technische Aufgabe löst das erfindungsgemäße Verfahren zum Elektroporieren eines biologischen Prozessgutes, indem das biologische einem niederfrequenten elektrischen Feld ausgesetzt wird, wobei das dem elektrischen Feld ausgesetzte biologische Prozessgut von einer Barriere umschlossen ist, die im Wesentlichen flüssigkeitsundurchlässig und für das elektrische Feld durchlässig ist.

Die erfindungsgemäße Vorrichtung zur Elektroporation von biologischem Prozessgut löst diese Aufgabe dadurch, dass eine Barriere zur Aufnahme des biologischen Prozessgutes in dem Behandlungsbereich angeordnet ist, wobei die Barriere im Wesentlichen flüssigkeitsundurchlässig und für das elektrische Feld durchlässig ist.

Die vorliegende Erfindung betrifft ferner ein elektroporiertes biologisches Prozessgut, das von einer Barriere umschlossen ist, die im Wesentlichen flüssigkeitsundurchlässig und für elektrische Felder durchlässig ist.

Es hat sich herausgestellt, dass für eine erfolgreiche Elektroporation mit einem niederfrequenten elektrischen Feld zwar ein gewisser Flüssigkeitsgehalt notwendig ist, um eine elektrische Leitfähigkeit zur Energieübertragung herzustellen. Aus Sicht der Wirksamkeit der Elektroporation ist überraschenderweise jedoch kein Wasserüberschuss erforderlich. Es wird in der Regel eine Menge an Flüssigkeit als Energieübertragungsmedium eingesetzt, die in diesem Maße nicht erforderlich ist.

Die vorliegende Erfindung löst das Problem möglicher Kontaminationen des biologischen Prozessguts oder des Prozessmediums, beispielsweise des Prozesswassers, durch den Einsatz einer Barriere. Die Barriere dient als Massentransportbarriere. Sie ist zwar für die elektrische Energie, also das elektrische Feld durchlässig, nicht aber für die Prozessflüssigkeit. Eine im Wesentlichen flüssigkeitsundurchlässige Barriere ist vollständig oder nur für eine festgelegte Menge an Flüssigkeit (pro festgelegter Fläche und Zeitintervall) durchlässig, die so gering ist, dass während der Elektroporation keine Flüssigkeit die Barriere passiert. Die Barriere stellt sicher, dass zwar die für die Elektroporation erforderliche Energie des niederfrequenten elektrischen Feldes passieren und auf das in der Barriere eingeschlossene biologische Prozessgut einwirken kann. Gleichzeitig unterbindet die Barriere einen unerwünschten Massentransport, der zu einer Kontamination des Prozesswassers durch Auslaugen oder Abwaschen von Produktbestandteilen des Prozessgutes oder umgekehrt einer Kontamination des Prozessgutes durch Inhaltsstoffe wie Verschmutzungen aus dem Prozessmedium führen kann. Auch eine nachteilige Befeuchtung oder Wasseraufnahme von Prozessgütern kann vermieden werden. Dies ist beispielsweise bei der Elektroporation von biologischen Prozessgütern mit geringem Wassergehalt von Vorteil, bei denen ein Befeuchten weitestgehend vermieden werden soll. Bei der Elektroporation von Saaten, Getreide oder blättrigen Produkten wie Tee oder Gras kann der benötigte Aufwand für eine anschließende Trocknung signifikant reduziert werden, wenn das erfindungsgemäße Verfahren eingesetzt wird. Zudem können Prozessgüter jeden Aggregatzustandes elektroporiert werden, weil die Barriere dem Prozessgut eine gewisse Formstabilität verleiht und ausschließt, dass es sich mit dem Prozessmedium vermischt. Auch die Elektroporation im Prozessmedium löslicher biologischer Prozessgüter wird mit dem der vorliegenden Erfindung ermöglicht.

Unter einem biologischen Prozessgut ist jede Art biologischer Zellen bzw. organischer Materialien, also Biomasse im weitesten Sinne, zu verstehen. Das erfindungsgemäße Verfahren kann zum Einsatz kommen bei der Bioenergiegewinnung für industrielle Zwecke, beispielsweise der Zuckergewinnung, Bioethanolherstellung, Ölgewinnung, Gewinnung ätherischer Öle, oder der Stärkegewinnung. Es kann zum Behandeln von Pflanzen oder Pflanzenteilen (zum Beispiel die Trocknung für die Saat, Gewürz-, Tee-, Tabak- oder Kräuterherstellung), oder für den Aufschluss pflanzlicher und tierischer Zellen bzw. die Herstellung von Nahrungsmitteln oder Nahrungsmittelkomponenten verwendet werden. Eine weitere mögliche Anwendung ist die Behandlung von Fleisch, zum Beispiel mit dem Ziel der Herstellung von Rohpökelware. Durch die Behandlung mit einem niederfrequenten elektrischen Feld kann eine Elektroporation und ein Aufschluss der tierischen Zellen erreicht werden, der zu einer beschleunigten Abtrocknung führt. Während der Elektroporation sollte eine Wasseraufnahme in das Prozessgut weitestgehend vermieden werden. Erst bei der anschließenden Reifung und Trocknung der Rohpökelware ist die Wasserabgabe wünschenswert. Ein weiteres mögliches Einsatzgebiet ist die Entkeimung des biologischen Prozessgutes, beispielsweise von Säften, Smoothies, Milchprodukten, Feinkostartikeln oder anderen Lebensmitteln und Nahrungsmitteln wie Obst oder Gemüse. Der Einsatz einer umschließenden Barriere verhindert eine Kontamination des Prozessgutes durch Verschmutzungen aus dem energieübertragenden Prozessmedium.

Die Barriere umschließt das biologische Prozessgut, wenn es dem elektrischen Feld ausgesetzt wird. Umschließen im Sinne der vorliegenden Erfindung bedeutet einen Einschluss des biologischen Prozessgutes dergestalt, dass eine Grenze zwischen dem energieübertragenden Prozessmedium einerseits und dem biologischen Prozessgut anderseits geschaffen wird.

Die Erfindung kann mit den folgenden, jeweils für sich vorteilhaften und beliebig miteinander kombinierbaren Weiterentwicklungen und vorteilhaften Ausgestaltungen weiter verbessert werden.

Bei dem Schritt des Elektroporierens kann das niederfrequente elektrische Feld ein nicht-thermisch wirkendes elektrisches Feld sein, bei dem die Energieobergrenze so bemessen ist, dass im Wesentlichen keine Erwärmung des Prozessgutes im Sinne einer ohmschen Erhitzung stattfindet. Dadurch wird vermieden, dass unerwünschte thermische Veränderungen des Gutes auftreten. Über die geeignete Wahl der Elektroporationsparameter, insbesondere Energieeintrag / Zeitintervall kann die Erwärmung kontrolliert werden. Ein nicht-thermisch wirkendes Feld ist dann vorhanden, wenn während der Elektroporation keine Temperatursteigerung des Prozessgutes von über 5 °C stattfinden.

Durch das elektrische Feld kann ein Energieeintrag von mindestens 1 kJ/kg in das biologische Prozessgut erfolgen. Ein Energieeintrag in dieser Größenordnung ist gut geeignet, um je nach Art des biologischen Prozessgutes eine irreversible Elektroporation, also eine dauerhafte Aufhebung der Semipermeabilität der Zellmembranen herzustellen. Je nach Einsatzzweck und Prozessgut kann der Energieeintrag ausgewählt werden. Soll mit der Elektroporation nur der Massentransport z. B. für eine bessere Trocknung oder Durchmischung von Substanzen im Prozessgut erfolgen, kann der Energieeintrag 2 kJ/kg bis 20 kJ/kg betragen. Bei Anwendungen zur Entkeimung und zum Abtöten von Mikroorganismen mittels Elektroporation kann der Energieeintrag höher, vorzugsweise bis 125 kJ/kg sein.

Gemäß einer Ausführungsform kann das biologische Prozessgut einem elektrischen Feld von 0,5 kV/cm bis 50 kV/cm ausgesetzt werden. Die Feldstärke kann beispielsweise im Bereich von 1kV/cm bis 10 kV/cm bei einem biologischen Prozessgut mit pflanzlichen oder tierischen Zellen liegen. Falls die Zellmembran von Einzellern oder Mikroorganismen in dem Prozessgut elektroporiert werden soll, kann eine Feldstärke von beispielsweise 5 kV/cm bis 30 kV/cm verwendet werden. Derartige Feldstärken sind nicht nur funktionell, sondern lassen sich einfach mit handelsüblichen industriellen Kondensatoren, beispielsweise Marx-Kondensatoren erzielen, und vermeiden, dass unerwünschte thermische Effekte bei der Elektroporation auftreten, die zu unerwünschten Änderungen in der Struktur bzw. chemischen Zusammensetzung des Prozessgutes führen.

Das niederfrequente elektrische Feld kann insbesondere ein gepulstes elektrisches Feld sein. So kann das biologische Prozessgut einem gepulsten elektrischen Feld ausgesetzt sein. Im Sinne einer Zeit- und Energieoptimierung kann das Prozessgut wenigstens 10 elektrischen Pulsen, vorzugsweise 10 bis 200 elektrischen Pulsen und besonders bevorzugt 30 bis 50 elektrischen Pulsen ausgesetzt sein.

Es hat sich überraschenderweise herausgestellt, dass besonders gute Ergebnisse erzielt werden, wenn das biologische Prozessgut einem niederfrequenten elektrischen Feld mit einer Frequenz von 1 Hz bis 1 kHz ausgesetzt wird. Dabei können elektrische Pulse im Mikro- bis Millisekundenbereich mit einer Rate von 1 Hz bis 1 kHz wiederholt werden.

Der Kondensator zur Erzeugung des niederfrequenten elektrischen Feldes kann bei einer beispielhaften Ausführungsform der erfindungsgemäßen Vorrichtung wenigstens zwei Elektroden umfassen, die mit einem Impulsgenerator verbunden sind. Als Impulsgenerator kann beispielsweise ein Hochspannungsimpulsgenerator eingesetzt werden, der niederfrequente elektrische Felder in Form von kurzen Pulsen einer Wiederholungsrate von unter 9 kHz, beispielsweise von 1 Hz bis 1 kHz, einer hohen Spannung im Kilovoltbereich erzeugt. Marx-Generatoren können als Hochspannungsimpulsgeneratoren eingesetzt werden. Im Kondensator können verschiedene Elektrodenformen zur Anwendung kommen. Platten-, Ring-, Gitter-, Hohl-, konische oder Durchflusselektroden sind möglich.

Die Barriere ist einerseits flüssigkeitsundurchlässig und andererseits für das elektrische Feld durchlässig. Die Durchlässigkeit von Materialien für elektrische Felder ist die Permittivität. Bei Permittivitätswerten handelt es sich um relative Werte, die zur elektrischen Feldkonstanten ε₋₀, der Permittivität für Vakuum in Relation gesetzt werden. Luft bildet dabei die Referenz und hat die Permittivität ε = 1. Eine Barriere gilt dann als für das elektrische Feld durchlässig im Sinne der vorliegenden Erfindung, wenn ihre relative Permittivität mindestens ε = 10 beträgt.

Gemäß einer Ausführungsform kann die Barriere fluiddicht oder semipermeabel sein. Fluiddicht bedeutet, dass weder Gase noch Flüssigkeiten die Barriere passieren können. Eine semipermeable Barriere ist flüssigkeitsdicht, also undurchlässig für Flüssigkeiten, aber gasdurchlässig. Eine semipermeable Barriere kann beispielsweise wasserdicht und wasserdampfdurchlässig, also atmungsaktiv sein. Eine semipermeable Barriere kann dann eingesetzt werden, wenn das elektroporierte Prozessgut anschließend im von der Barriere umschlossenen Zustand getrocknet werden soll. Fluiddichte Barrieren können gleichzeitig als Endverpackung des Prozessgutes verwendet werden. Eine Kontamination oder Oxidation des Produktes nach der Elektroporation, beispielsweise während des Handels oder sogar des Lagerns bis zum Endkunden, kann dadurch vermieden werden.

Es können sowohl synthetische als auch natürliche Barrieren zum Umschließen des Prozessgutes eingesetzt werden, welche flüssigkeitsdurchlässig sind und eine geeignete elektrische Leitfähigkeit aufweisen. Natürliche Barrieren bestehen aus natürlichen Polymeren beispielsweise Polysacchariden, Proteinen, Nukleinsäuren, also Polymeren im Allgemeinen, die von Organismen gebildet werden. Dazu zählen unter anderem Kollagene oder Alginate, aber auch Zellulosen oder andere Naturfasern.

Synthetische Barrieren können aus einem elektrisch leitfähigen Kunststoff bestehen. Dies kann ein Polymer wie ein Polyamid, Polyethylen, Polyetheretherketon, Polyoxymethylen sein, dem leitfähige Substanzen, beispielsweise Graphite oder Metalle zugesetzt sind.

Die Barriere kann aus Biokunststoff bestehen. Unter Biokunststoffe fallen biobasierte Polymere, also organische Verbindungen, die modifiziert werden um Polymere herzustellen. Dies können beispielsweise Polylactide, Polyhydroxyalkanoate, Polyhydroxybutyrate, Thermoplaste auf Ligninbasis oder Epoxyacrylate auf Basis von Ölen wie Lein- oder Palmöl sein. Auch biologisch abbaubare Polymere werden zu Biokunststoffen gezählt. Zu biologisch abbaubaren Polymere gehören diverse erdölbasierte Kunststoffe, beispielsweise bestimmte Polyester, Polyvinylalkohol, Polybutylenadipat-terephthalat, Polybutylensuccinate, Polycaprolactone oder Polyglycolide.

Gemäß einer weiteren Ausführungsform kann die Barriere auf das biologische Prozessgut aufgebracht werden. Das Prozessgut kann beispielsweise mit dem die Barriere bildenden Material beschichtet werden. Zum Beschichten kann das Prozessgut mit einem die Barriere bildenden Material besprüht, damit extrudiert oder in ein solches Material eingetaucht werden.

Das biologische Prozessgut kann auch in die Barriere eingebracht werden, so dass die Barriere das Produkt umhüllt. Denkbar sind hier Barrieren in Form von Folien, Hüllen oder anderen Verpackungen. Unter einer Hülle ist ganz allgemein ein Gegenstand zu verstehen, der einen Raum bildet, welcher das biologische Material umschließt und somit das Prozessmedium und das Prozessgut räumlich voneinander abgrenzt. Zu Hüllen zählen Behälter im weitesten Sinne. Behälter sind also jede Art von Gefäß und Gehäuse, Verpackungen, Membranen, Häute oder sonstige Umhüllungen.

Gemäß einer Ausführungsform kann das Prozessgut in die Barriere eingebracht werden, die eine Folie, ein Schlauch, eine Hülle oder Verpackung ist. Eine andere Möglichkeit ist, dass die Barriere in einem Tauchverfahren oder einer Extrusion auf das biologische Produkt aufgebracht wird. Zum Beschichten kann die Barriere auch aufgesprüht werden. Dabei bietet es sich an, auf übliche Methoden bzw. Abfülltechniken, beispielsweise Schlauchbeutelmaschinen, Tauchverfahren oder Extrusion bzw. Koextrusionen zurückzugreifen. Um die Umhüllung zu verschließen, kann das umhüllende Material beispielsweise mit einer Siegelnaht oder einem Zip verschlossen werden oder mit einer aseptischen Kupplung versehen sein.

Gemäß einer weiteren Ausführungsform kann das biologisch Prozessgut nach der Elektroporation aus der Barriere entnommen werden. Im Falle einer Hülle, beispielsweise einer Verpackung als Barriere kann das Prozessgut aus dieser herausgenommen werden. Eine Beschichtung als Barriere kann nach dem Elektroporieren durch geeignete Maßnahmen entfernt werden. Die Barriere kann beispielsweise durch Bestrahlung oder Wärme zersetzt werden.

Gemäß einer weiteren Ausführungsform kann die Barriere konfektioniert werden um die gewünschten Eigenschaften einer Endverpackung aufzuweisen. Derartige Eigenschaften sind beispielsweise eine ausreichende UV-Barriere oder eine Bedruckbarkeit. Die Barriere kann ein- oder mehrschichtig sein, wobei zumindest eine Schicht, die flüssigkeitsundurchlässig und für das elektrische Feld durchlässig ist, das Prozessgut während des Elektroporierens umschließt.

Gemäß einer weiteren Ausführungsform kann die Barriere nach dem Elektroporieren als Produktverpackung verwendet werden. Sie kann beispielsweise als Transportverpackung eingesetzt werden. Eine solche Transportverpackung kann bis zu einer weiteren Verwendung wie einem Ab- oder Umfüllen genutzt werden. Die Barriere kann auch dauerhaft als Endverpackung benutzt werden. Sofern die Barriere, welche das Prozessgut während des Elektroporierens umschließt, noch nicht alle gewünschten Eigenschaften einer Endverpackung aufweist, kann die Barriere nach dem Elektroporieren von einer Verpackungsschicht umhüllt werden. Die zusätzliche Umhüllung kann zusätzliche Eigenschaften wie eine bessere Bedruckbarkeit, eine UV-Barriere oder bessere Dauerbeständigkeit bereitstellen. Die Verpackungsschicht kann durch Umhüllen, Besprühen oder Tauchen aufgebracht werden. Auch im Falle einer umhüllenden Verpackungsschicht kann auf übliche Abfülltechniken wie ein Tauchverfahren oder eine Extrusion zurückgegriffen werden.

Eine Ausführungsform der erfindungsgemäßen Vorrichtung kann vorsehen, dass die Barriere eine den Behandlungsbereich durchdringende Transportstrecke abgrenzt. Das Prozessgut kann dabei entlang der Transportstrecke durch den Behandlungsbereich bewegt werden und bleibt aufgrund der Barriere vom umgebenden Prozessmedium abgegrenzt. Es kann beispielsweise ein Schlauch als Barriere im Behandlungsbereich vorgesehen sein, durch den das Prozessgut während der Elektroporation gefördert wird. Anstelle eines Schlauches kann selbstverständlich auch ein formstabiler Tunnel als Barriere eingesetzt werden. Wichtig ist, dass die Barriere den Behandlungsabschnitt unterteilt und das Prozessgut vom umgebenden Prozessmedium deutlich trennt.

Anstelle einer aktiven Förderung des Prozessgutes durch ein Fördermedium, beispielsweise eine Pumpe oder ein Förderband, kann auch ein Fallrohr, Falltrichter oder Fallschlauch eingesetzt werden, durch welchen das Prozessgut mittels Schwerkraft bewegt wird.

Die oben unter Bezugnahme auf das Verfahren dargestellten vorteilhaften Ausgestaltungen der Elektroporation sowie der Barriere beziehungsweise der Konfektionierungsschritte sind selbstverständlich in analoger Weise entsprechend auf die erfindungsgemäße Vorrichtung beziehungsweise das erfindungsgemäße elektroporierte biologische Prozessgut übertragbar. Die erfindungsgemäße Vorrichtung kann beispielsweise eine Umschließungsstufe aufweisen, welche dem Kondensator vorgeschaltet und die ausgestaltet ist, das biologische Prozessgut mit einer Barriere zu umschließen. Die erfindungsgemäße Vorrichtung kann ferner eine Konfektionierungsstufe aufweisen, welche dem Kondensator vorzugsweise nachgelagert und die ausgestaltet ist, die Barriere mit einer Verpackungsschicht zu umhüllen.

Im Folgenden wird die Erfindung anhand vorteilhafter Ausgestaltungen mit Bezug auf die Zeichnungen beispielhaft näher erläutert. Die dabei dargestellten vorteilhaften Weiterentwicklungen und Ausgestaltungen sind jeweils voneinander unabhängig und können beliebig miteinander kombiniert werden, je nachdem, wie im Anwendungsfall notwendig ist.

Es zeigen:
- Fig. 1: eine schematische Darstellung einer ersten Ausführungsform der vorliegenden Erfindung;
- Fig. 2: eine schematische Darstellung einer Ausführungsform zum Umschließen eines Prozessgutes mit einer Barriere;
- Fig. 3: eine schematische Querschnittsansicht einer weiteren Ausführungsform der vorliegenden Erfindung;
- Fig. 4: ein schematischer Längsschnitt der Ausführungsform aus Fig. 3;
- Fig. 5: ein Fließdiagramm einer weiteren Ausführungsform der vorliegenden Erfindung und
- Fig. 6: ein Fließdiagramm einer weiteren Ausführungsform der vorliegenden Erfindung.

Nachfolgend werden beispielhafte beispielhafte Verfahren und Vorrichtungen zum Elektroporieren eines biologischen Prozessgutes 1 präsentiert.

Das Verfahren zum Elektroporieren eines biologischen Prozessgutes 1 beinhaltet, dass das Prozessgut 1 elektroporiert wird, indem es einem niederfrequenten elektrischen Feld ausgesetzt wird, wobei das dem elektrischen Feld ausgesetzte Prozessgut 1 von einer Barriere 2 umschlossen ist. Die Barriere 2 ist flüssigkeitsundurchlässig und für das elektrische Feld durchlässig. Die Vorrichtung zum Elektroporieren des biologischen Prozessgutes 1 umfasst einen Kondensator 3 zur Erzeugung eines niederfrequenten elektrischen Feldes in einem Behandlungsbereich 4, wobei die Barriere 2 zur Aufnahme des biologischen Prozessgutes 1 in dem Behandlungsbereich 4 angeordnet ist, und wobei die Barriere 2 flüssigkeitsundurchlässig und für das elektrische Feld durchlässig ist.

Der Kondensator 4 (im Folgenden auch Elektroporator genannt) umfasst wenigstens zwei Elektroden 5, die den Kondensator 4 zur Erzeugung des niederfrequenten elektrischen Feldes ausbilden. Die Elektroden 5 sind über Energieleitungen 6 mit einer Spannungsquelle 7 verbunden. In der gezeigten Ausführungsform sind die beiden Elektroden 5 parallel zueinander angeordnet und bilden zwischen sich den Behandlungsbereich 4 aus. Konkret sind die beiden Elektroden 5 an sich gegenüberliegenden Seiten einer Behandlungskammer 8 angebracht. Auf diese Weise kann in der Behandlungskammer 8, genauer deren Behandlungsbereich 4 ein homogenes elektrisches Feld zur gleichmäßigen Behandlung des Prozessgutes 1 und effektiven Elektroporation erzeugt werden.

Als Spannungsquelle 7 kann ein Impulsgenerator 9 eingesetzt werden, mit dem elektrische Impulse einer hohen Spannung im Kilovoltbereich und einer kurzer Dauer im Mikro- bis Millisekundenbereich einer Niederfrequenz, also von weniger als 9 kHz, vorzugsweise von 1 Hz bis 1 kHz generiert werden können. Die Elektroden 5 können aus Edelstahl oder einer Titanlegierung gefertigt sein.

In der Behandlungskammer 8 ist ein Prozessmedium 10, hier eine Flüssigkeit enthalten. Das flüssige Prozessmedium 10 sorgt dafür, dass im Behandlungsbereich 4 zwischen den beiden Elektroden 5 ein elektrisches Feld erzeugt und dessen Energie übertragen werden kann. Im Unterschied zu beispielsweise Mikrowellen sind niederfrequente elektrische Felder in einem Dielektrikum nicht übertragbar.

Durch die übertragene Energie des elektrischen Feldes wird das Prozessgut elektroporiert, was zu einem erhöhten Massentransport in das bzw. aus dem Prozessgut führt. Die Elektroporation bewirkt allerdings auch, dass unerwünschte Inhaltsstoffe aus dem Prozessgut 1 in das Medium 10 austreten und/oder Verschmutzungen aus dem Prozessmedium 10 in das Prozessgut 1 eindringen. Zudem nimmt ein elektroporiertes Prozessgut 1, solange es sich in dem flüssigen Prozessmedium 10 befindet, Flüssigkeit auf und wird durchfeuchtet, was nicht immer gewollt ist. Bei der Behandlung von Saaten, Tee oder Samen kann eine Elektroporation beispielsweise erwünscht sein, um deren Trocknungseigenschaften zu beeinflussen bzw. bestimmte Vorgänge, wie enzymatische Reaktionen zu initiieren. Gleichzeitig ist eine Flüssigkeitsaufnahme in das Prozessgut nachteilig, da die aufgenomme Flüssigkeit anschließend wieder entfern werden muss, was einen Zusatzaufwand bei der Trocknung bedeutet. Um diese unerwünschten Prozesse zu vermeiden, umschließt die Barriere 2 das Prozessgut 1.

Die Barriere kann sowohl fluiddicht, also sowohl für Flüssigkeiten als auch Gase undurchlässig, als auch semipermeabel, also flüssigkeitsdicht und gasdurchlässig sein.

In der gezeigten Ausführungsform wird eine Verpackung 11 als Barriere 2 eingesetzt. In diese Verpackung 11 wurde das Prozessgut 1, beispielsweise mehrere Weizenkörner, eingebracht. Im verpackten Zustand wird das Prozessgut im Behandlungsbereich elektroporiert, wobei die Barriere 2 verhindert, dass es zu unerwünschtem Massentransport zwischen Prozessmedium 10 und Prozessgut 1 kommt. Die Verpackung 11 kann entweder formstabil, beispielsweise eine Box oder ein sonstiger Behälter, oder forminstabil, beispielsweise ein Schlauch, ein Beutel oder eine Folie, sein. Um den mit Gas gefüllten Leerraum innerhalb der Verpackung 11 möglichst gering zu halten, kann die Verpackung 11 beispielsweise eine Vakuumverpackung sein, die eng an dem verpackten Prozessgut 1 anliegt und so gut wie gar kein ein Dielektrikum bildendes Leervolumen enthält.

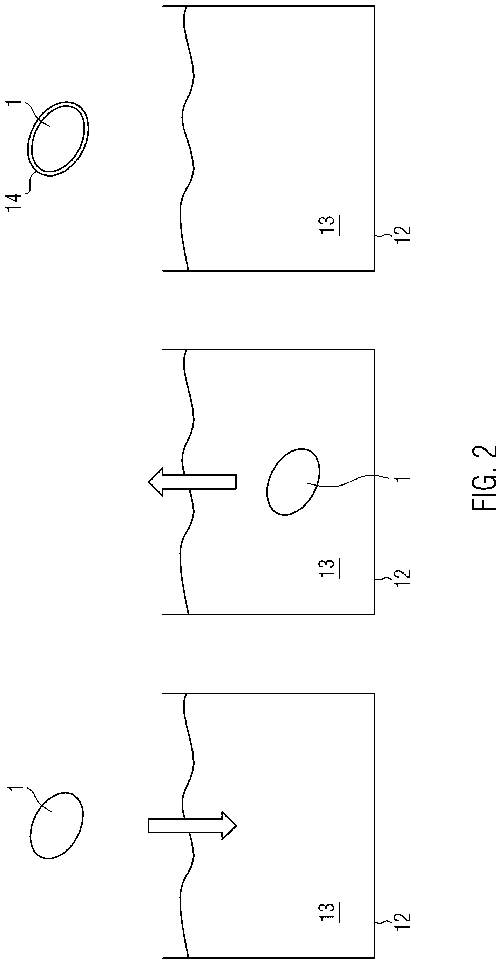

Anstatt das Prozessgut 1 in eine Verpackung 11 einzubringen, kann, wie nachfolgend unter Bezugnahme auf Fig. 2 erläutert werden wird, auch eine Barriere 2 auf das biologische Prozessgut 1 aufgebracht werden.

Das in Fig. 2 gezeigte Beispiel sieht vor, dass die Barriere 2 in einem Tauchverfahren auf das Prozessgut 1 aufgebracht wird. Dazu kann ein Tauchbad 12 vorgesehen sein, das mit einer Beschichtungsflüssigkeit 13 befüllt ist.

Zum Beschichten wird das Prozessgut 1 in die im Tauchbad 12 befindliche Beschichtungsflüssigkeit 13 eingetaucht und solange einwirken gelassen, bis sich eine Beschichtung 14 als Barriere um das Prozessgut 1 bildet. Die Beschichtung 14 umschließt das Prozessgut 1 vollständig, auch nachdem es aus dem Tauchbad 12 entnommen worden ist.

Eine Beschichtung 14 ist von Vorteil, weil diese Art der Barriere 2 unmittelbar auf der Oberfläche des Prozessgutes 1 gebildet wird. Man vermeidet mit Gas gefüllte Hohlräume, welche die Effektivität der Elektroporation herabsetzen, da sie ein Dielektrikum darstellen. Zudem ist eine Beschichtung 14 als Barriere 2 flexibel einsetzbar und unabhängig von der körperlichen Form des Prozessgutes 1. Dies kann dann von Vorteil sein, wenn das Prozessgut 1 unregelmäßige Formen aufweist und es schwierig ist, eine formstabile Verpackung zu finden, welche zu jeder Form des Prozessgutes 1 passt.

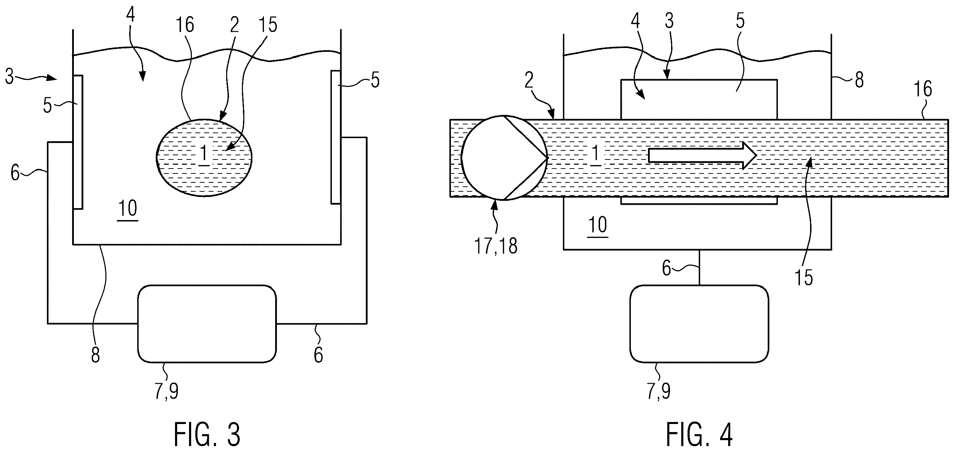

In den Figuren 3 und 4 ist eine weitere Ausführungsform des erfindungsgemäßen Verfahrens und der erfindungsgemäßen Vorrichtung 20 gezeigt. Diese Ausführungsform ist für die Elektroporation eines fließfähigen Prozessgutes 1 geeignet. Bei Elementen, deren Funktion und/oder Aufbau identisch zu Elementen der vorher beschriebenen Ausführungsformen ist, werden nachfolgend dieselben Bezugszeichen verwendet.

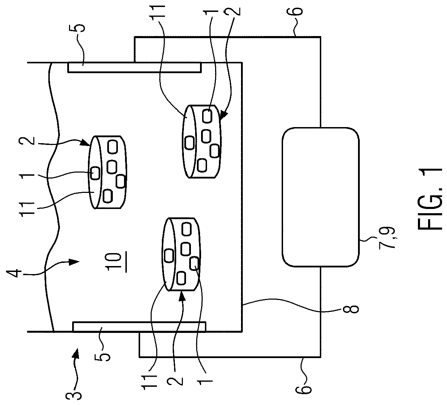

Der Elektroporator / Kondensator 3 und die Behandlungskammer 8 der Ausführungsform aus den Figuren 3 und 4 sind nahezu identisch mit denen der Fig. 1. In der Behandlungskammer 8 ist ein flüssiges Prozessmedium 10 enthalten und parallel an gegenüberliegenden Seiten angeordnete Elektroden 5 bilden den Kondensator 3. Die Elektroden 5 sind über Energieleitungen 6 mit einer Spannungsquelle 7 verbunden, sodass im Behandlungsberich 4 zwischen den Elektroden 5 ein elektrisches Feld erzeugbar ist.

In der Ausführungsform der Figuren 3 und 4 grenzt die Barriere 2 eine den Behandlungsbereich durchdringende Transportstrecke 15 ab. Die Transportstrecke 15 kann ein Rohr, ein Tunnel, oder wie in der gezeigten Ausführungsform ein Transportschlauch 16 sein, der sich von einem Ende der Behandlungskammer 8 durch den zwischen den Elektroden 5 gebildeten Behandlungsbereich 4 hindurch bis zum gegenüberliegenden Ende der Behandlungskammer 8 erstreckt.

Durch den Transportschlauch 16 kann ein fließfähiges Prozessgut 1 gefördert werden. Hierzu kann ein Förderelement 17, beispielsweise eine Fluidpumpe 18 vorgesehen sein, mittels derer das Prozessgut 1 innerhalb des Transportschlauchs 16 durch den Behandlungsbereich 4 bewegt und dabei elektroporiert wird.

Selbstverständlich sind auch andere Ausgestaltungen denkbar. Beispielsweise kann ein Förderband zum Transport von Stückgütern vorgesehen sein, mittels welchem das von einer Barriere 2 umschlossene Prozessgut 1, beispielsweise ein in einer Verpackung 11 aufgenommenes Prozessgut durch den Behandlungsbereich 4 bewegt und dabei elektroporiert wird.

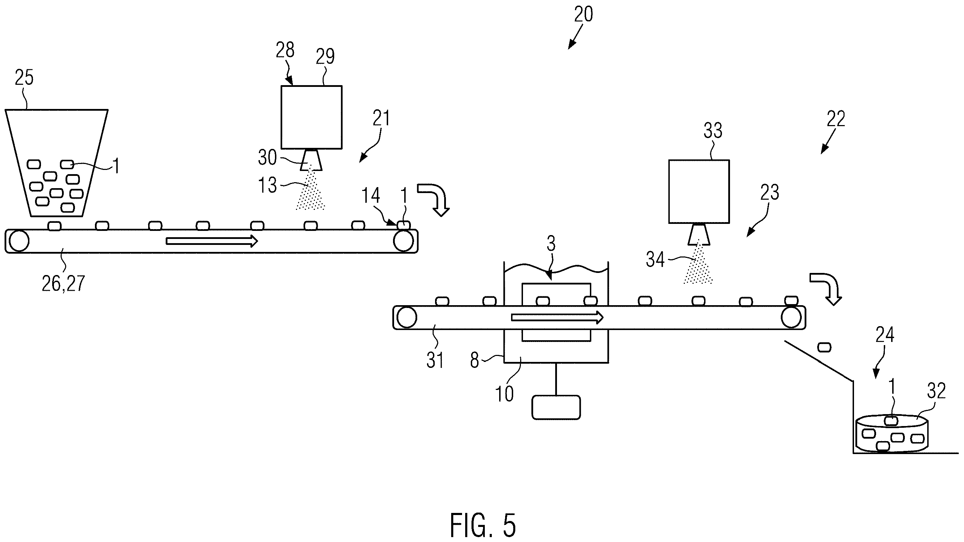

Nachfolgend wird auf eine weitere Ausführungsform unter Bezugnahme auf die Fig. 5 eingegangen.

Die Vorrichtung 20 zur Elektroporation des biologischen Prozessgutes 1 umfasst eine Umschließungsstufe 21, einen Elektroporator / Kondensator 3, der sich an die Umschließungsstufe 21 anschließt. Dem Kondensator 3 nachgelagert ist eine Konfektionierungsstufe 22, die in der gezeigten Ausführungsform zweistufig mit einem Entschichtungsbereich 23 und einer Endverpackungsstufe 24 versehen ist.

Die Umschließungsstufe 21 beinhaltet eine Zuführung 25 zum Aufgeben des Prozessgutes 1 sowie eine Transportvorrichtung 26, beispielsweise ein Transportband 27. Über die Zuführung 25 werden die einzelnen Stücke des Prozessgutes 1 auf das Transportband 27 aufgebracht und von diesem entlang der Pfeilrichtung zum anderen Ende des Transportbandes 27 gefördert.

Über dem Transportband 27 ist in der Umschließungsstufe 21 eine Beschichtungsvorrichtung 28 angeordnet. In der gezeigten Ausführungsform besteht die Beschichtungsvorrichtung 28 aus einem Behälter 29, der eine Beschichtungsflüssigkeit 13 enthält. An der dem Transportband 27 zugewandten Seite ist der Behälter 29 mit einer Sprühdüse 30 versehen, mittels welcher die Beschichtungsflüssigkeit 13 auf das auf dem Transportband 27 geförderte Prozessgut 1 appliziert wird und eine umschließende Beschichtung 14 als Barriere 2 bildet. Die Sprühbeschichtung ist in Fig. 5 nur schematisch und beispielhaft dargestellt. Es können auch mehrere Sprühdüsen 30 vorgesehen sein, welche von mehreren Seiten das Prozessgut 1 mit Beschichtungsflüssigkeit 13 besprühen. Es kann anstelle eines Behälters 29 mit Sprühdüse 30 auch ein Tauchbad 12 (in Fig. 5 nicht gezeigt) vorgesehen sein. Die Umschließungsstufe 21 könnte auch eine Verpackungsvorrichtung aufweisen, beispielsweise eine Schlauchbeutelmaschine, um das Prozessgut 1 in die Barriere 2 einzubringen und darin zu umschließen. Auch ein Extruder könnte zum Beschichten verwendet werden.

Das nun umschlossene Prozessgut 1 wird am Ende des Transportbandes 27 an den Kondensator 3 zur Elektroporation übergeben. Der Kondensator 3 wird, in analoger Weise zu den Ausführungen der Fig. 1 und 3, von zwei parallel angeordneten Elektroden 5 gebildet. Die Elektroden 5 sind an sich gegenüberliegenden Innenwänden einer Behandlungskammer 8 angeordnet. In der Behandlungskammer 8 befindet sich ein Prozessmedium 10, um die niederfrequente Energie des Kondensators 3 zu übertragen und das Prozessgut 1 zu elektroporieren, wenn es durch den zwischen den Elektroden 5 gebildeten Behandlungsbereich 4 bewegt wird.

Die Ausführungsform der Fig. 5 sieht dazu eine Behandlungskammer 8 mit Kondensator 3 vor, die ähnlich zu denen der Figuren 3 und 4 ausgebildet ist. Im Unterschied zu den Figuren 3 und 4 ist in Fig. 5 kein Transportschlauch 16, sondern ein weiteres Förderband 31 vorgesehen ist, welches das Prozessgut 1 durch den Behandlungsbereich 4 bewegt.

In Fig. 5 schließt sich an den Kondensator 3 die Konfektionierungsstufe 22 an. Diese Konfektionierungsstufe 22 beinhaltet zunächst einen Entschichtungsbereich 23 zum Entfernen der Beschichtung 14 und abschließend eine Endverpackungsstufe 24.

Der Entschichtungsbereich 23 ist in der gezeigten Ausführungsform ähnlich zu der Umschließungsstufe 21 aufgebaut. Er umfasst einen weiteren Behälter 33, von dem aus ein Entschichtungsmittel 34 auf das beschichtete Prozessgut 1 einwirken und die Beschichtung 14 entfernen kann. Das Entschichtungsmittel 34 kann eine bestimmte Strahlung sein, welche die Beschichtung 14 auflöst. Es kann Wärme sein, welche die Beschichtung 13 thermisch zersetzt, oder es kann ein sonstiges beispielsweise chemisches Agens als Entschichtungsmittel 34 eingesetzt werden.

Danach wird das elektroporierte und nun ausgepackte, also nicht mehr beschichtete Prozessgut 1 am Ende des Förderbandes 31 der Endverpackungsstufe 24 über eine weitere Übergabe zugeführt und in eine Endverpackung 32 eingebracht. Die Endverpackung 32 umhüllt das Prozessgut 1 mit einer weiteren Verpackungsschicht 35. Eine solche zusätzliche Verpackungsschicht 35 kann zusätzliche Eigenschaften, beispielsweise eine bessere Bedruckbarkeit, eine UV-Barriere oder bessere Dauerbeständigkeit bereitstellen. Zusätzlich ist es möglich, mehrere Prozessgüter in einer Endverpackung 32 zu platzieren.

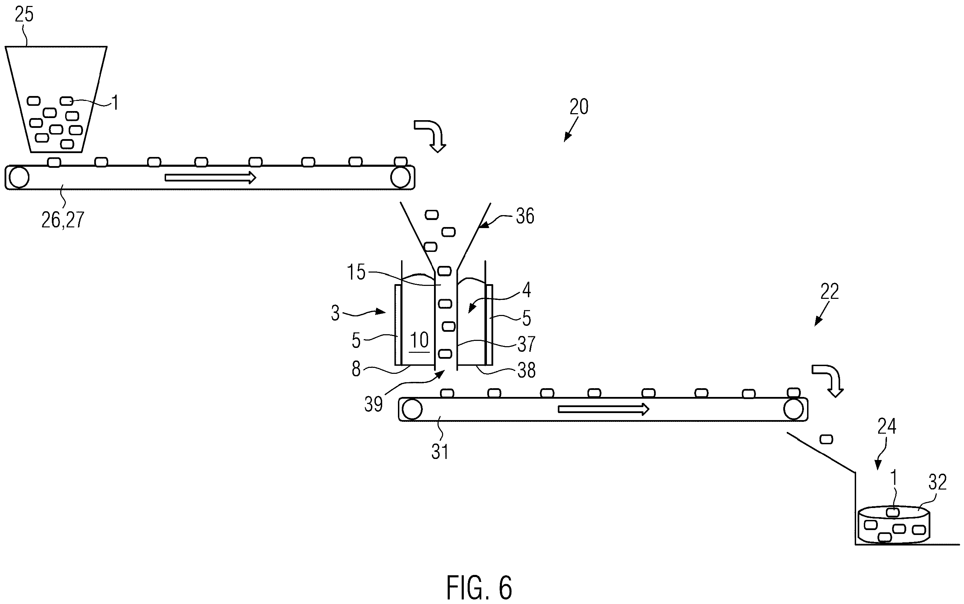

Abschließend wird auf eine weitere Ausführungsform unter Bezugnahme auf die Fig. 6 eingegangen. Die Ausführungsform der in Fig. 6 gezeigten Vorrichtung 20 entspricht in Teilen der Ausführungsform aus Fig. 5.

Es ist eine Zuführung 25 vorhanden, mittels welcher das Prozessgut 1 dem Kondensator 3 zugeführt wird. Im Unterschied zur Fig. 5 ist bei der Ausführungsform der Fig. 6 keine Umschließungsstufe 21, also kein Behälter 29 mit Sprühdüse 30, und kein Entschichtungsbereich 23 vorgesehen.

Stattdessen wird das Prozessgut 1 innerhalb eines Falltrichters 36 vom Transportband 27 zum Förderband 31 geführt. Am weiten Ende des Falltrichters 36 findet die Übergabe von Transportband 27 zu Falltrichter 36 statt. Der schmale Auslauf 37 des Falltrichters 36 bildet eine den Behandlungsbereich 4 durchdringende Transportstrecke 15. In Fig. 6 ist erneut eine Behandlungskammer 8 vorgesehen, welche vom Aufbau her der Behandlungskammer aus Fig. 1 entspricht.

Der Auslauf 37 des Falltrichters 36 tritt durch die Oberfläche des Prozessmedium 10 ein, erstreckt sich zwischen den Elektroden 5 im Behandlungsbereich 4 und tritt durch den Boden 38 der Behandlungskammer 8 aus dieser aus. Das offene Ende 39 des Auslaufs 37 endet oberhalb des Förderbandes 31.

In der in Fig. 6 gezeigten Ausführungsform bildet der Falltrichter 36, konkret dessen Auslauf 37, somit die Barriere 2, welche das Prozessgut 1 im Behandlungsbereich umschließt und vom Prozessmedium 10 abgrenzt.

Den Abschluss der Vorrichtung 20 aus Fig. 6 bildet die Endverpackungsstufe 24. Diese entspricht der Endverpackungsstufe 24 der in Fig. 5 gezeigten Ausführungsform.

Das erfindungsgemäße Verfahren und die erfindungsgemäße Vorrichtung 20 vermeiden die unerwünschten Nachteile einer Elektroporation, beispielsweise eine Kontamination des Prozessmediums 10 und/oder des Prozessgutes 1 sowie eine unerwünschte Befeuchtung oder Wasseraufnahme. Dadurch erschließt die vorliegende Erfindung neue Einsatzzwecke für die Elektroporation. Bei der Behandlung von Prozessgütern mit geringem Wassergehalt kann beispielsweise ein Befeuchten weitestgehend vermieden werden, was beispielsweise bei Saaten, Getreide oder blättrigen Produkten wie Tee oder Tabak von Interesse ist und einen unnötigen Aufwand für die Trocknung beziehungsweise ein Auslaugen vermeidet. Bei der Behandlung von Fleisch, beispielsweise mit dem Ziel der Herstellung von Rohpökelware, kann während der Elektroporation eine Wasseraufnahme weitestgehend verhindert werden. Im Rahmen der abschließenden Reifung und Trocknung der Rohpökelware wird die wünschenswerte Wasserabgabe durch die Elektroporation dagegen begünstigt. Auch bei durch Elektroporation entkeimten Prozessgütern wie beispielsweise Säften, Smoothies oder auch Milchprodukten, Feinkostartikeln, Obst oder Gemüse kann durhc vorliegenden Erfindung eine Rekontamination vermieden werden.

### Bezugszeichen

- 1: Prozessgut
- 2: Barriere
- 3: Kondensator
- 4: Behandlungsbereich
- 5: Elektroden
- 6: Energieleiter
- 7: Spannungsquelle
- 8: Behandlungskammer
- 9: Impulsgenerator
- 10: Prozessmedium
- 11: Verpackung
- 12: Tauchbad
- 13: Beschichtungsflüssigkeit
- 14: Beschichtung
- 15: Transportstrecke
- 16: Transportschlauch
- 17: Förderelement
- 18: Pumpe
- 20: Vorrichtung
- 21: Umschließungsstufe
- 22: Konfektionierungsstufe
- 23: Entschichtungsbereich
- 24: Endverpackungsstufe
- 25: Zuführung
- 26: Transportvorrichtung
- 27: Transportband
- 28: Beschichtungsvorrichtung
- 29: Behälter
- 30: Sprühdüse
- 31: Förderband
- 32: Endverpackung
- 33: Behälter
- 34: Entschichtungsmittel
- 35: Verpackungsschicht
- 36: Falltrichter
- 37: Auslauf
- 38: Boden
- 39: Offenes Ende

## Patentansprüche

1. Verfahren zum Elektroporieren eines biologischen Prozessgutes (1), wobei das biologische Prozessgut (1) elektroporiert wird, indem es einem niederfrequenten elektrischen Feld ausgesetzt wird, und wobei das dem elektrischen Feld ausgesetzte biologische Prozessgut (1) von einer Barriere (2) umschlossen ist, die im Wesentlichen flüssigkeitsundurchlässig und für das elektrische Feld durchlässig ist.

2. Verfahren nach Anspruch 1, wobei durch das elektrische Feld ein Energieeintrag von wenigstens 1 kJ/kg in das biologische Prozessgut (2) erfolgt.

3. Verfahren nach Anspruch 1 oder 2, wobei das biologische Prozessgut (1) einem elektrischen Feld von 0,5 kV/cm bis 50 kV/cm ausgesetzt wird.

4. Verfahren nach einem der Ansprüche 1 bis 3, wobei das biologische Prozessgut (1) einem gepulsten elektrischen Feld ausgesetzt wird.

5. Verfahren nach einem der Ansprüche 1 bis 4, wobei das biologische Prozessgut (1) einem niederfrequenten elektrischen Feld von 1 Hz bis 1 kHz ausgesetzt wird.

6. Verfahren nach einem der Ansprüche 1 bis 5, wobei die Barriere (2) fluiddicht oder semipermeabel ist.

7. Verfahren nach einem der Ansprüche 1 bis 6, wobei die Barriere (2) aus einem elektrisch leitfähigen Kunststoff, einem natürlichen Polymer oder aus einem Biokunststoff besteht.

8. Verfahren nach einem der Ansprüche 1 bis 7, wobei die Barriere (2) auf das biologische Prozessgut (1) aufgebracht wird, und/oder das biologische Prozessgut (1) in die Barriere (2) eingebracht wird.

9. Verfahren nach Anspruch 8, wobei das biologische Prozessgut (1) in die Barriere (2) eingebracht wird, die eine Folie, ein Schlauch, eine Hülle oder eine Verpackung (11) ist, und/oder die Barriere (2) in einem Tauchverfahren oder einer Extrusion auf das biologische Prozessgut (1) aufgebracht wird.

10. Verfahren nach einem der Ansprüche 1 bis 9, wobei das biologische Prozessgut (1) nach der Elektroporation aus der Barriere (2) entnommen wird.

11. Verfahren nach einem der Ansprüche 1 bis 10, wobei die Barriere (2) ein- oder mehrschichtig ist.

12. Verfahren nach einem der Ansprüche 1 bis 11, wobei die Barriere (2) nach dem Elektroporieren als Produktverpackung verwendet wird.

13. Verfahren nach einem der Ansprüche 1 bis 12, wobei die Barriere (2) nach dem Elektroporieren von einer Verpackungsschicht (35) umhüllt wird.

14. Vorrichtung (20) zur Elektroporation eines biologischen Prozessgutes (1), umfassend einen Kondensator (3) zur Erzeugung eines niederfrequenten elektrischen Feldes in einem Behandlungsbereich (4), wobei eine Barriere (2) zur Aufnahme des biologischen Prozessgutes (1) in dem Behandlungsbereich (4) angeordnet ist, und wobei die Barriere (2) im Wesentlichen flüssigkeitsundurchlässig und für das elektrische Feld durchlässig ist.

15. Vorrichtung (20) gemäß Anspruch 14, wobei die Barriere (2) eine den Behandlungsbereich (4) durchdringende Transportstrecke (15) abgrenzt.
